# EUROPÄISCHE PATENTSCHRIFT

(11) **EP 0 786 086 B1**
(45) Veröffentlichungstag und Bekanntmachung des Hinweises auf die Patenterteilung: **08.12.1999**
(21) Anmeldenummer: 95933318.8
(22) Anmeldetag: 30.09.1995
(51) Int. Cl.: G01N 33/543, C12N 11/14, C07K 17/14, G01N 33/552

(54) **VERFAHREN ZUR IMMOBILISIERUNG VON BIOMATERIAL AUF EINER UNTERLAGE**
PROCESS FOR IMMOBILIZING BIO-MATERIAL ON A SUBSTRATE
PROCEDE D'IMMOBILISATION D'UN BIOMATERIAU SUR UN SUBSTRAT

(30) Priorität: 08.10.1994 DE 4435998
(43) Veröffentlichungstag der Anmeldung: 30.07.1997
(73) Patentinhaber: FORSCHUNGSZENTRUM JÜLICH GMBH, 52425 Jülich (DE)
(72) Erfinder: THUST, Marion, D-50939 Köln (DE); SCHÖNING, Michael, Josef, D-52428 Jülich (DE); VETTER, Joachim, D-50678 Köln (DE); KAUPP, Ulrich, Benjamin, D-52062 Aachen (DE); KORDOS, Peter, D-52428 Jülich (DE); LÜTH, Hans, D-52076 Aachen (DE)
(86) Internationale Anmeldenummer: DE9501373
(87) Internationale Veröffentlichungsnummer: WO9611403

(56) Entgegenhaltungen:
- EP-A- 0 350 073
- US-A- 5 234 820
- JOURNAL OF MOLECULAR CATALYSIS, Bd. 43, Nr. 3, - 1988 AMSTERDAM NL, Seiten 293-301, XP 000562703 E. TAMIYA ET AL. 'Characterization of immobilized urease membrane on silicon nitride layer.' in der Anmeldung erwähnt
- ABSTRACTS OF PAPERS OF THE AMERICAN CHEMICAL SOCIETY, - 7.September 1986 ANAHEIM CA USA, Seite 13 XP 000563391 T. YANG ET AL. 'Covalent immobilization of immunoglobulin to polystyrene and silican nitride chips'

## Beschreibung

Die Erfindung bezieht sich auf ein Verfahren zur Immobilisierung von Biomaterial auf einer Unterlage mit Si₃N₄-Oberfläche, an die das Biomaterial über Bindungsvermittler kovalent gebunden ist.

Die Immobilisierung von Biomaterial ist für die Ausnutzung von Bioaktivität, insbesondere bei Flüssigkeitskontakt, von erheblicher Bedeutung. Sie spielt sowohl in der Verfahrenstechnik als, auch in der Analytik bei Trennoperationen oder für die wiederkehrende Ausnutzung der Biofunktion eine große Rolle. Weitere Interessengebiete sind im Pharma- und Medizinbereich sowie in der Umwelttechnik zu sehen.

Bekannt sind zahlreiche Versuche zur Immobilisierung von Biomaterial an unterschiedlichen Oberflächen, auch mineralischer Art wie Glas, das im allgemeinen zunächst silanisiert wird.

Von E. Tamiya et al. wird im J. Mol. Catal. 43 (1988) 293-301 eine Immobilisierung von Urease an einem mit dünner Silberschicht versehenen Quarzkristall beschrieben, auf dessen Oberfläche eine Siliciumnitridschicht aufgesputtert wird. Der so behandelte Kristall wurde 24 h an Lufi gelagert, gewaschen und im Luftbad getrocknet. Danach erfolgte eine Bedampfung mit y-Amino-propyl-triethoxysilan, gefolgt von einer Bedampfung mit Glutaraldehyd. Der so erzeugte dünne organische Oberflächenfilm von ca. 100 Å Dicke wird als wenig porös bezeichnet. Eine Verknüpfung von Silan und Aldehyd konnte nicht richtig beobachtet werden.

Das aus wäßriger Lösung am Film angelagerte Enzym hatte eine Aktivität relativ zur freien Urease von nur 2,25 %. Dieses lmmobilisierungskonzept erscheint nicht voll befriedigend.

Gegenstand der Erfindung ist eine neue Art der Biomaterial-Fixierung, die mit einer geringen Zahl von Behandlungsschritten auskommt und auf reproduzierbare Weise zu relativ beständigen Produkten führt.

Das erfindungsgemäße Verfahren zur Immobilisierung von Biomaterial auf einer Unterlage der eingangs genannten Art ist im wesentlichen dadurch gekennzeichnet, daß man auf der Immobilisierungsunterlage eine Si₃N₄-Oberfläche mit bindungsaktiven NHₓ-Gruppen vorsieht, mit denen ein heterobifunktioneller Crosslinker mit NH2-reaktiver Aldehyd-, Ester-, Halogenid-, Epoxid-, Imin- oder Isocyanatgruppe einerseits und einer biomaterial-reaktiven Gruppe andererseits zur Reaktion gebracht wird, an den dann das Biomaterial angekoppelt wird.

Weitere Besonderheiten der Erfindung ergeben sich aus den Patentansprüchen.

Die Erfindung ist für die Immobilisierung von Enzymen, Mikroorganismen, Zellen, Antikörpern, Antigenen, Organellen, Gewebeschnitten usw. auf Unterlagen wie Halbleitersubstraten, Folien, Wandflächen, Granulaten, Bauteilen, insbesondere mineralischer Art, geeignet und in Anwendungsbereichen, wie eingangs genannt, nützlich.

Siliciumnitrid-Oberflächen können u.a. durch CVD-Technik aus einem SiH₄/NH₃-Gemisch abgeschieden werden (siehe A. Garde et al. ESSDERC 1994 - 11.-15. Sept. 1994 Ed. C. Hill & P. Ashburn). Sie nehmen an Luft Sauerstoff auf und neigen bei Einwirkung von Feuchtigkeit zur Hydrolyse unter Bildung von Si-OH, Si-NH und Si-NH₂ Gruppen. Diese Gruppen können als reaktive Funktionen für die Ankupplung von Biomaterial über Crosslinker an die Nitridoberfläche ausgenutzt werden.

Besonders zweckmäßig wird beim erfindungsgemäßen Verfahren eine durch Behandlung mit verdünnter Flußsäure von Oxiden befreite Si₃N₄-Oberfläche benutzt und in zwei Schritten für eine kovalente Enzym-Ankopplung gesorgt, indem ein Crosslinker gewählt wird, dessen eine Funktion zunächst mit den NH₂-Gruppen oder auch NH-Gruppen der Nitridoberfläche reagiert und dessen zweite Funktion nachfolgend mit dem Protein umgesetzt wird. Alternativ kann der Crosslinker auch zuerst mit dem Protein umgesetzt, und das Produkt dann mit der Si₃N₄-Oberfläche zur Reaktion gebracht werden.

Als NH₂-reaktive Gruppen des Crosslinkers bieten sich insbesondere Aldehyd-, Halogenid-, Epoxid-, Imid- oder Isocyanatfunktionen an. Eine Vielzahl von Reaktionsmöglichkeiten mit Aminogruppen findet sich im übrigen z.B. in der US-PS 5 234 820. Für die Praxis werden bereits unterschiedliche Verbindungen von der Fa. Pierce im "Immuno Technology Catalog & Handbook" von 1992/3 angeboten.

Für die Umsetzung mit dem Biomaterial werden Crosslinker-Funktionen ausgenutzt, die zu einer kovalenten Bindung über funktionelle Gruppen des Enzyms befähigt sind, insbesondere mit terminalen Carboxy-Gruppen oder Seitenkettengruppen, wie -SH, -COOH oder -OH Gruppen oder aromatischen Ringen. Erfindungsgemäß wird die Anbindung des Biomaterials an die Nitridoberfläche mithin unter Ausnutzung eines heterobifunktionellen Crosslinkers angestrebt, wobei als heterobifunktioneller Crosslinker hier auch ein solcher Crosslinker verstanden werden soll, der zwei grundsätzlich chemisch gleichartige Funktionen aber mit unterschiedlicher Reaktivität gegenüber den unterschiedlichen Umsetzungspartnern aufweist.

Die heterobifunktionellen Crosslinker werden schrittweise mit der Si₃N₄-Oberfläche und dann mit dem Protein umgesetzt. Die nachfolgend im einzelnen angegebenen aminospezifischen Reaktionen laufen bei Raumtemperatur in neutralem bis leicht alkalischem pH-Wert ab. Erhöhung sowohl der Temperatur als auch des pH-Werts steigert die Reaktionsgeschwindigkeit, jedoch auch die Hydrolyserate des Crosslinkers. Der benutzte Puffer sollte weder Amine noch andere Verbindungen enthalten, mit denen die funktionellen Gruppen des Crosslinkers reagieren könnten.

N-Hydroxysuccinimidester reagiert spezifisch mit primären Aminen. Unter Abspaltung von N-Hydroxysuccinimid entsteht eine Amidbindung zwischen dem primären Amin und der Restgruppe des verwendeten Esters. Sofern kein wasserlösliches Analogon verwendet wird, muß ein Crosslinker, der diese funktionnelle Gruppe besitzt zuerst in einer kleinen Menge eines organischen Lösungsmittels (z. B. DMSO) gelöst und erst dann auf die Endkonzentration in wäßrigem Puffer verdünnt werden. Die Ionenstärke des Puffers sollte nicht zu hoch sein um Aussalzungseffekte zu vermeiden. Ein leicht alkalischer pH-Wert (7-9) garantiert, daß sich die primären Amine in unproptoniertem Zustand befinden.

Aldehyde verfügen über eine stark reduzierende Carbonylgruppe. Diese reagiert mit primären Aminen unter Wasserabspaltung.

Die Reaktion von primären Aminen mit Imidoestern läuft im pH-Bereich zwischen 8 und 9 ab. Der Ester wird dabei abgespalten und das primäre Amin bildet mit der Imidogruppe eine Guanidinoverbindung.

Die Anbindung an das Protein geschieht nun mithilfe der zweiten noch freien funktionellen Gruppe des Crosslinkers. Diese kann spezifisch oder unspefisch mit Thiol-, Carboxyl- oder Carbohydratgruppen des Proteins reagieren.

Besitzt der Crosslinker als zweites funktionelles Ende eine thiolspezifische Gruppe wie Maleimid ein aktiviertes Halogenid oder Pyridyldisulfid, so muß das zu bindende Protein eine freie Sulfhydrylgruppe(üblicherweise von einem Cysteinrest) besitzen. Sollte diese nicht verfügbar sein. so kann sie durch Reduktion von Proteindisufiden generiert werden. Alternativ können primäre Amine des Proteins so modifiziert werden, daß Sulfhydrylgruppen zur Verfügung stehen (Trauts Reagenz). Um Oxidation dieser Gruppen zu vermeiden, muß verwendete Puffer entgast werden. Zugabe des Komplexbildners EDTA verhindert die Oxidation durch etwaige in der Lösung vorhandene Metalle.

Maleimid reagiert in leicht saurem bis neutralem Milieu (pH 6,5-7,5), während für Halogenide und Pyridyldisulfid pH-Werte größer oder gleich 7 zu empfehlen sind.

Glycolysierte Proteine können über die Hydroxygruppe an den Zuckerseitenketten vernetzt werden. Wird ein carbohydrataktiver Crosslinker benutzt, der als funktionelle Gruppe z. B. ein Hydrazid besitzt, so muß die Carbohydratgruppe des Proteins zunächst zu einem Aldehyd oxidiert werden (z. B. mit NaIO₄). Die entstehende Carbonylgruppe reagiert dann mit dem Hydrazit zu Semicarbazon.

Eine Möglichkeit der direkten Verknüpfung zwischen Carboxy- und Aminogruppen besteht durch die Reaktion mit Carbodiimiden. Im saurem pH-Bereich (4-5) überführen Carbodiimide die Carboxygruppe in eine aktivierte Esterbindung.Diese reagiert mit primären Aminen unter Ausbildung einer Amidbindung und Abspaltung von Harnstoff.

Bei Verwendung eines Crosslinkers, dessen nicht-aminospezifisches funktionelles Ende eine photoaktivierbare Gruppe (z. B. Azidophenyl) aufweist, muß die gesamte Immobilisierung in einer Dunkelkammer unter Rotlicht durchgeführt werden. Die Azidogruppe von Azidophenyl wird durch Lichteinstrahlung der Wellenlänge 265-275 nm aktiviert.

Die Erfindung ist für die Anbindung unterschiedlichster Biomaterialien an Unterlagen oder Träger allgemeinster Art brauchbar. Sie wurde speziell am Beispiel von Penicillinsensoren getestet, weshalb sich die nachfolgende Beschreibung auf solche bezieht. Dabei wird auf die angefügten Bezeichnungen Bezug genommen. Diese zeigen schematisch:
- **Fig. 1**: ein Sensorprinzip (Meßanordnung),
- **Fig. 2**: eine typische Meßkurve für einen Konzentrationsbereich von 10⁻⁴ bis 10⁻¹ Mol/l Penicillin und
- **Fig. 3**: die Kalibrierungskurve eines erfindungsgemäßen Sensors.

### Beispiel:

Auf p- bzw. p⁺-dotierten Siliciumwafern (1 mOhmcm - 30 Ohmcm) wurde zuerst eine elektrisch nicht leitende Schicht von Siliciumdioxid mit einer Dicke von 5 - 100 nm durch thermische Trockenoxidation zwischen 700 und 1200 °C (hier bei 1000 °C) in einem Diffusionsofen erzeugt. Darauf wurde ebenfalls nicht leitendes Siliciumnitrid mit einer Dicke von 10-100 nm durch chemische Abscheidung in der Gasphase (PECVD) aufgebracht. Das Verhältnis SiH₄/NH₃ im Reaktionsgas betrug 2/l, die Substrattemperatur 200 - 500 °C (hier 300 °C) und der Druck während der Abscheidung 1 - 3 Torr (hier 1,5 Torr). Es folgte ein Temperschritt unter N₂-Atmosphäre (5-60 Minuten bei 700-1000°C). Schließlich wurde die unpolierte Substratseite mit einem Ohmschen Kontakt (z. B. 10 - 1000 nm Al, Au) versehen. Das verwendete Material wurde durch thermisches Aufdampfen im Vakuum bei einem Basisdruck < 10⁻⁵ mbar aufgebracht. Die Abscheidungsrate lag zwischen 0,1 und 10 nm/s. Anschließend wurde der Wafer in einem RTA-Ofen bei 150 - 500 °C (hier 400 °C) in N₂-Atmosphäre getempert.
Unmittelbar vor Beginn des Enzymimmobilisierungsprozesses wurden die Wafer in Aceton, 2-Propanol und destilliertem Wasser im Ultraschallbad gereinigt und 10 - 60 s (hier 30 s) in verdünnter Flußsäure (1-10% HF) geätzt.
Bei Verwendung des heterobifunktionellen Crosslinkers ANB-NOS (N-5-Azido-2-nitrobenzoyloxysuccinimide) wurde dieser zunächst in einer kleinen Menge DMSO gelöst und dann mit 0,2 M Triethanolaminpuffer (pH 5-9) auf eine Endkonzentration von 0,5-10 mM verdünnt. Diese Lösung wurde auf die Si₃N₄-Oberfläche aufgebracht und zwischen 5 und 40 Minuten lang bei Raumtemperatur inkubiert. Bei niedrigerer Temperatur muß länger inkubiert werden. Nicht an die Siliciumnitridoberfläche gebundene Moleküle wurden durch Abspülen mit Triethanolaminpuffer (TEA) entfernt. Danach wurde das Enzym (Penicillinase Typ 1 aus Bacillus Cereus, Sigma P 0389) in einem Puffer, der keine Aminogruppen enthält (z. B. TEA, insbesondere nicht TRIS- oder Glycinpuffer) gelöst (1000-5000 Units/ml) und auf die mit Crosslinker vorbehandelte Siliciumnitridoberfläche gegeben. Nach einer Inkubationzeit von 1-240 min (hier 15 min) bei Temperaturen zwischen 4 und 60 °C, insbesondere bei Raumtemperatur wurde die Anbindung der Enzymmoleküle an die noch freie funktionnelle Gruppe des Crosslinkers durch Licht im Wellenlängenbereich von 320-350 nm induziert. Nach Abschluß des Immobilisierungsverfahrens wurden die einsatzfähigen Penicillinsensoren mit 0,1 M TRIS-Puffer (pH 7-8) und destilliertem Wasser gespült und mindestens 10 Minuten an Luft bzw. unter N₂- oder Inertgasatmosphäre getrocknet.

Mit den auf diese Weise hergestellten Feldeffektsensoren wurden Messungen zur Bestimmung der Penicillinkonzentration in wäßrigen Lösungen durchgeführt.

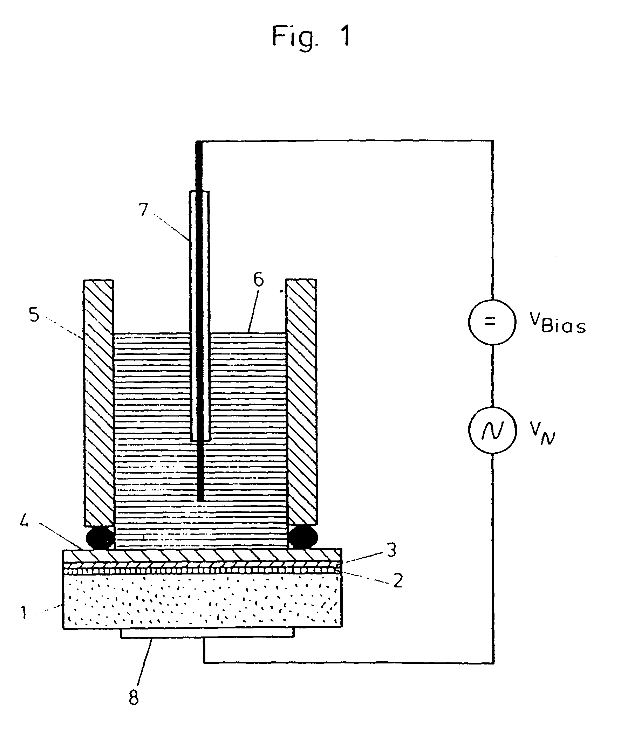

In **Fig. 1** ist die Meßanordnung schematisch dargestellt. Der erfindungsgemäß hergestellte Feldeffektsensor bestehend aus dem Siliciumsubstrat 1, der Isolatorschicht 2 (Siliciumdioxid und Silciumnitrid), der Crosslinkerschicht 3 und der Enzymschicht (Penicillinschicht) 4 wurden in eine Meßzelle integriert. Die Meßzelle wurde mit einer wäßrigen Meßlösung befüllt, die Penicillin G in einer Konzentration zwischen 10⁻⁵ und 1 Mol/l enthielt. In die Meßlösung 6 taucht eine Referenzelektrode (z. B. Ag/AgCl) 7 ein. Die Potentiale werden über die Referenzelektrode 7 und eine Kontaktelektrode 8 am Siliciumsubstrat abgegriffen.

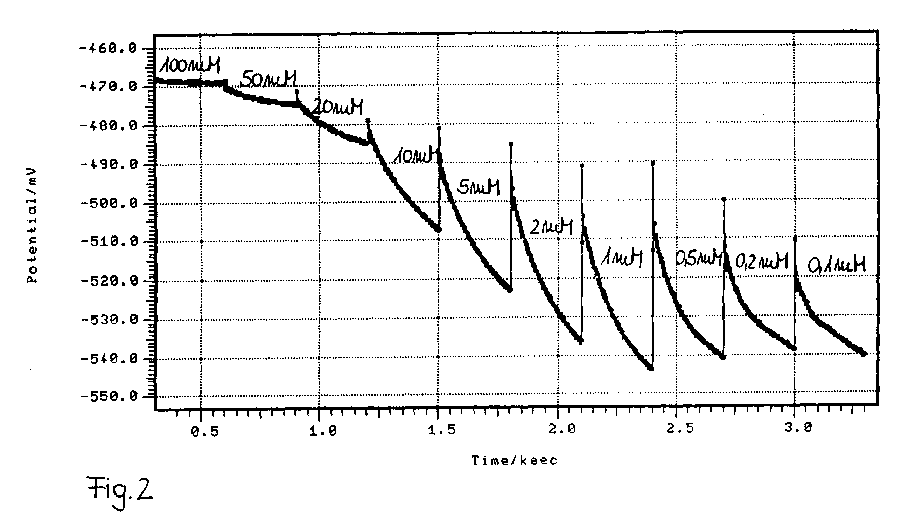

In **Fig. 2** ist eine typische Meßkurve dargestellt, die im CONCAP (CONstant CAPacitance) - Modus im Konzentrationsbereich zwischen 10⁻⁴ und 10⁻¹ Mol/l Penicillin aufgenommen wurde. Penicillin G Natriumsalz (Sigma P 3032) wurde dazu in 10 mM TRIS-HCI Puffer, pH 7 gelöst. Mit steigender Penicillinkonzentration steigt die Konzentration der gebildeten Penicilloinsäure und somit die Konzentration der Wasserstoffionen in unmittelbarer Nähe der als pH-Transduktor wirkenden Siliciumnitridfläche. Dies hat eine Verschiebung des Potentials an der Grenzfläche Siliciumnitrid/Elektolyt zu positiveren bzw. negativeren Spannungswerten hin zur Folge. Die Auftragung über der Zeit erlaubt eine Beobachtung des konzentrationsabhängigen Potentialverlaufs der Enzymreaktion. Zu gekennzeichneten Zeiten fand der Wechsel der Meßlösungen statt.

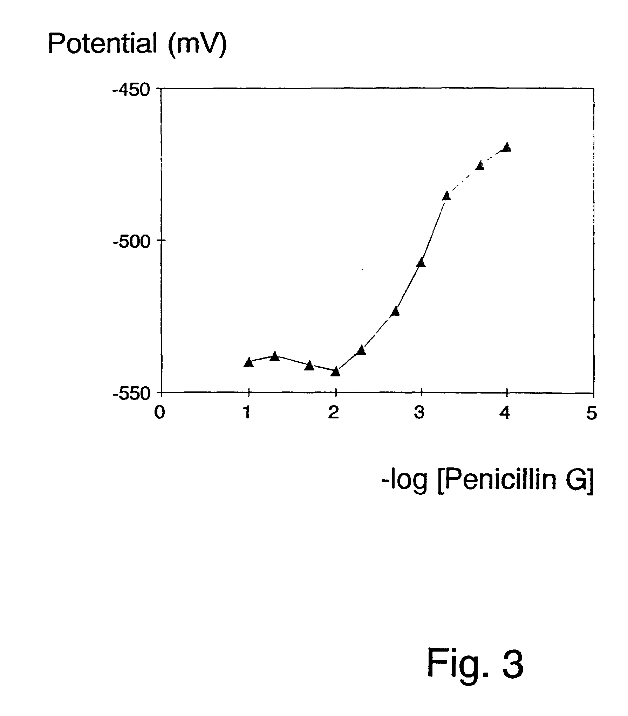

**Fig. 3** zeigt die aus **Fig. 2** ermittelte chemische Übertragungskennlinie. Sie stellt die Kalibrierungskurve des erfindungsgemäß hergestellten Feldeffektsensors dar. Angaben über die Sensitivität eines potentiometrischen Chemo- oder Biosensors werden im Hinblick auf den zugrundeliegenden Nernstschen Zusammenhang im linearen Bereich dieser Kurve, d. h. in dem Bereich. in dem ein logarithmischer Zusammenhang zwischen der Penicillinkonzentration und dem anliegenden Potential besteht gemacht. Dieser Bereich liegt im erfindungsgemäß hergesteliten Sensor zwischen pPenicillin 2,3 und 3,3, was 0,5 und 5 mM entspricht. Die Empfindlichkeit beträgt 50 mV pro Dekade.

Die exakte Lage des linearen Meßbereichs und die absolute Empfindlichkeit hängen wesentlich von der Wahl der Pufferzusammensetzung, dessen Konzentration, d. h. der Pufferkapazität und dem pH Wert ab. Durch geeignete Wahl dieser Parameter kann der für eine Messung erforderliche Meßbereich gezielt eingestellt werden. Beispielsweise liegt der lineare Meßbereich bei Verwendung von IMIDAZOL-Puffer (pH 7) zwischen 2 und 20 mM Penicillin, fur HEPES-Puffer etwa zwischen 1 und 10 mM. Erhöhung des pH-Werts verschiebt die Lage der linearen Bereiche der Kalibrationskurven zu höheren Penicillinkonzentrationen hin, Erniedrigung entsprechend zu niedrigeren.

Die erfindungsgemäß hergestellten Sensoren weisen eine hohe Langzeitstabilität von mehr als 140 Tagen auf. Die Empfindlichkeit liegt bei 50 mV pro Penicillindekade.

Die Herstellung eines Feldeffekttransistors, der im Gatebereich einen gleichartigen Aufbau wie die in der Erfindung beschriebene kapazitive Schichtstruktur aufweist ist möglich.

## Patentansprüche

1. Verfahren zur Immobilisierung von Biomaterial auf einer Unterlage mit Si₃N₄-Oberfläche, an die das Biomaterial über Bindungsvermittler kovalent gebunden wird,
**dadurch gekennzeichnet.**
daß man auf der Immobilisierungsunterlage eine Si₃N₄-Oberfläche mit bindungsaktiven NHₓ-Gruppen vorsieht, mit denen ein heterobifunktioneller Crosslinker mit NH2-reaktiver Aldehyd-, Ester-, Halogenid-, Epoxid-, Imin-oder Isocyanatgruppe einerseits und einer biomaterial-reaktiven Gruppe andererseits zur Reaktion gebracht wird, an den dann das Biomaterial angekoppelt wird.

2. Verfahren nach Anspruch 1,
**dadurch gekennzeichnet,**
daß die am biomaterialreaktive Gruppe oder Funktion des Crosslinkers eine mit terminalen oder Seitenkettenfunktionen von Proteinen reagierende Gruppe ist.

3. Verfahren nach Anspruch 1 oder 2,
**dadurch gekennzeichnet,**
daß ein Crosslinker mit einer mit Carboxyl-, Sulfhydryl- oder Hydroxygruppen reagierenden oder an aromatische Ringe ankuppelnden biomaterial-aktiven Gruppe gewählt wird.

4. Verfahren nach einem der vorangehenden Ansprüche
**dadurch gekennzeichnet,**
daß auf der Immobilisierungsunterlage eine 10 - 1000 nm dicke Si₃N₄-Schicht durch CVD aus einem SiH₄/NH₃-Gemisch abgeschieden und durch hydrolysierende Oberflächenreinigung mit bindungsaktiven NHₓ-Gruppen versehen wird.

5. Verfahren nach einem der vorangehenden Ansprüche,
**dadurch gekennzeichnet,**
daß als Biomaterial Enzyme, Mikroorganismen, Zellen, Antikörper, Antigene, Organellen oder Gewebeschnitte auf Halbleitersubstraten, Folien, Wandflächen oder Granulaten insbesondere mineralischer Art fixiert werden.

6. Verfahren in Abwandlung von Anspruch 1,
**dadurch gekennzeichnet,**
daß man für die Anbindung mittels des Crosslinkers diesen zunächst mit dem Biomaterial zur Reaktion bringt und danach für die Verknüpfung mit der Si₃N₄-Oberfläche sorgt.

## Claims

1. A method of immobilising biomaterial on a support having an Si3N4 surface to which the biomaterial is covalently bonded via bonding agents,
characterised in that
an Si₃N₄ surface which comprises NHₓ groups which exhibit active bonding properties is provided on the immobilisation support, which NHₓ groups are reacted with a hetero-bifunctional crosslinker which firstly comprises an aldehyde, ester, halide, epoxide, imine or isocyanate group which is reactive towards NH₂ and which secondly comprises a group which is reactive with biomaterial, to which crosslinker the biomaterial is then coupled.

2. A method according to claim 1,
characterised in that
the group or function of the crosslinker which reacts with biomaterial is a group which reacts with terminal or side chain functions of proteins.

3. A method according to claims 1 or 2,
characterised in that
a crosslinker is selected which comprises a biomaterial-active group which reacts with carboxyl, sulfhydryl or hydroxy groups or which couples to aromatic rings.

4. A method according to any one of the preceding claims,
characterised in that
an Si₃N₄ layer of thickness 10 - 1000 nm is deposited on the immobilisation support by CVD from an Si₃N₄/NH₃ mixture and is provided with NHₓ groups which exhibit active bonding properties by means of a hydrolysing surface cleaning step.

5. A method according to any one of the preceding claims,
characterised in that
enzymes, microorganisms, cells, antibodies, antigens, organelles or tissue sections are fixed as the biomaterial on semiconductor substrates, on foils, on wall areas or on granular materials, particularly those of a mineral nature.

6. A method as a modification of claim 1,
characterised in that
to effect bonding by means of the crosslinker the latter is first reacted with the biomaterial and binding to the Si₃N₄ surface is thereafter ensured.

## Revendications

1. Procédé d'immobilisation d'un biomatériau sur un substrat avec une surface de Si₃N₄ sur laquelle le biomatériau est lié par covalence au moyen d'un agent de liaison, caractérisé en ce qu'on prévoit, sur le substrat d'immobilisation, une surface de Si₃N₄ avec des groupes liants NHₓ avec lesquels on fait réagir un agent de réticulation hétérobifonctionnel ayant un groupe aldéhyde, ester, halogénure, époxyde, imino ou isocyanate apte à réagir avec NH₂, d'une part, et ayant un groupe apte à réagir avec un biomatériau, d'autre part, avec lequel le biomatériau est ensuite couplé.

2. Procédé selon la revendication 1, caractérisé en ce que le groupe ou la fonction de l'agent de réticulation apte à réagir avec le biomatériau est un groupe qui réagit avec des fonctions terminales ou de chaînes latérales de protéines.

3. Procédé selon la revendication 1 ou 2, caractérisé en ce qu'on choisit un agent de réticulation ayant un groupe réagissant avec des groupes carboxyle, sulfhydryle ou hydroxy ou un groupe apte à réagir avec un biomatériau et à coupler avec des noyaux aromatiques.

4. Procédé selon l'une des revendications précédentes, caractérisé en ce qu'il est prévu sur le substrat d'immobilisation une couche de Si₃N₄ de 10 à 1000 nm d'épaisseur déposée par CVD à partir d'un mélange de SiH₄/NH₃ et par nettoyage hydrolysant de la surface avec des groupes liants NHₓ.

5. Procédé selon l'une des revendications précédentes, caractérisé en ce qu'on fixe, comme biomatériau, des enzymes, des micro-organismes, des cellules, des anticorps, des antigènes, des organules ou des coupes de tissus sur des substrats semiconducteurs, des feuilles métalliques, des surfaces de parois ou des granules, en particulier de nature minérale.

6. Procédé modifié selon la revendication 1, caractérisé en ce que, pour la liaison au moyen de l'agent de réticulation, on fait d'abord réagir celui-ci avec le biomatériau et ensuite, on veille à l'enchaînement avec la surface de Si₃N₄.
